Europäisches Patentamt

European Patent Office

Office européen des brevets

(11) Publication number: **0 234 599 B1**

# EUROPEAN PATENT SPECIFICATION

(45) Date of publication of patent specification: **11.03.92**

(51) Int. Cl.[5]: **C12N 15/10**, C12N 15/24, C12P 21/02, A61K 37/02, C12N 1/20, G01N 33/50, A61K 49/00, //(C12N1/20, C12R1:19)

(21) Application number: **87103350.2**

(22) Date of filing: **13.10.83**

(60) Publication number of the earlier application in accordance with Art.76 EPC: **0 109 748**

The file contains technical information submitted after the application was filed and not included in this specification

(54) Cysteine-depleted muteins of interleukin-2, their preparation, formulations containing them, and genes, vectors and organisms suitable for use in the preparation of said muteins.

(30) Priority: **19.10.82 US 435154**
**15.04.83 US 486162**

(43) Date of publication of application:
**02.09.87 Bulletin 87/36**

(45) Publication of the grant of the patent:
**11.03.92 Bulletin 92/11**

(84) Designated Contracting States:
**AT BE CH DE FR GB IT LI LU NL SE**

(56) References cited:
**EP-A- 0 136 489**

**NATURE, vol. 294, 10th December 1981, pages 563-565, Macmillan Journals Ltd; H.M. SHEPARD et al.: "A single amino acid change in IFN-beta1 abolishes its antiviral activity"**

(73) Proprietor: **CETUS CORPORATION**
**1400 Fifty-Third Street**
**Emeryville California 94608(US)**

(72) Inventor: **Mark, David F.**
**217 Standbridge Ct.**
**Danville California 94526(US)**
Inventor: **Lin, Leo S.**
**40880 Los Pinos**
**Fremont California 94539(US)**
Inventor: **Yu Lu, Shi-Da**
**366 Euclid Avenue No. 109**
**Oakland California 94610(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN(GB)**

## Description

This invention is in the general area of recombinant DNA technology. More specifically it relates to mutationally altered biologically active Interleukin-2 proteins that differ from their parent analog by substitution of cysteine residues.

Biologically active proteins that are microbially produced via recombinant DNA (rDNA) technology may contain cysteine residues that are nonessential to their activity but are free to form undesirable intermolecular or intramolecular links.

Thus, human IL-2 is reported to have three cysteine residues located at positions 58, 105, and 125 of the protein. IL-2 is in an aggregated oligomeric form when isolated from bacterial cells and has to be reduced with reducing agents in order to obtain a good yield from bacterial extracts. In addition, the purified reduced IL-2 protein is unstable and readily reoxidized upon storage to an oligomeric inactive form. The presence of three cysteines means that upon reoxidation, the protein may randomly form one of three possible intramolecular disulfide bridges, with only one of those being the correct bridge as found in the native molecule.

The present invention is directed to producing by directed mutagenesis techniques mutationally altered biologically active IL-2 proteins (such proteins are called "muteins", Glossary of Genetics and Cytogenetics, 4th Ed. p 381, Springer-Verlag (1976)) that retain the activity of their parent analogs but lack the ability to form intermolecular links or undesirable intramolecular disulfide bonds. In this regard Shepard, H.M., et al, Nature (1981) 294:563-565 describe a mutein of IFN-$\beta$ in which the cysteine at position 141 of its amino acid sequence (there are three cysteines in native human IFN-$\beta$ at positions 17, 31, And 141, Gene (1980) 10:11-15 and Nature (1980) 285:542-547) is replaced by tyrosine. This mutein was made by bacterial expression of a hybrid gene constructed from a partial IFN-$\beta$ cDNA clone having a G→A transition at nucleotide 485 of the IFN-$\beta$ gene. The mutein lacked the biological activity of native IFN-$\beta$ leading the authors to conclude that the replaced cysteine was essential to activity.

Directed mutagenesis techniques are well known and have been reviewed by Lather, R.F. and Lecoq, J.P. in Genetic Engineering Academic Press (1983) pp 31-50. Oligonucleotide-directed mutagenesis is specifically reviewed by Smith, M. and Gillam, S. in Genetic Engineering: Principles and Methods, Plenum Press (1981) 3:1-32.

The invention provides a biologically active recombinant mutein of human interleukin-2, which mutein is incapable of forming an improper intramolecular disulfide bridge and is stable upon storage relative to bacterially derived reduced native interleukin-2, wherein the cysteine residue normally present at position 125, numbered in accordance with native interleukin-2, that is free to form a disulfide link and is non-essential to said bioligical activity has been substituted by a neutral amino acid, said mutein being a threonine$_{125}$interleukin-2 mutein, glycine$_{125}$interleukin-2 mutein, a valine$_{125}$interleukin-2 mutein, a leucine$_{125}$interleukin-2 mutein, and isoleucine$_{125}$interleukin-2 mutein, a histidine$_{125}$interleukin-2 mutein, a tyrosine$_{125}$interleukin-2 mutein, a phenylalanine$_{125}$interleukin-2 mutein, a tryptophan$_{125}$interleukin-2 mutein, or a methionine$_{125}$interleukin-2 mutein.

Another aspect of the invention is genes having DNA sequences that have been specifically designed ("designer genes") to encode the above described synthetic muteins. Other aspects of this aspect are expression vectors that include such designer genes, E. coli transformed with such vectors, and the use of such vectors in making synthetic muteins by expressing said vectors.

Another aspect of the invention is a method of making a DNA sequence as defined above comprising:

(a) hybridizing a single-stranded DNA comprising a strand of a gene that encodes for an interleukin-2 protein with a mutant oligonucleotide primer that is complementary to a region of said strand that includes the codon for said cysteine residue or the antisense triplet paired with said codon, as the case may be, except for a mismatch with said codon or said antisense triplet which mismatch defines a triplet that codes for said neutral animo acid;

(b) extending the primer with DNA polymerase to form a mutational heteroduplex; and

(c) replicating said mutational heteroduplex.

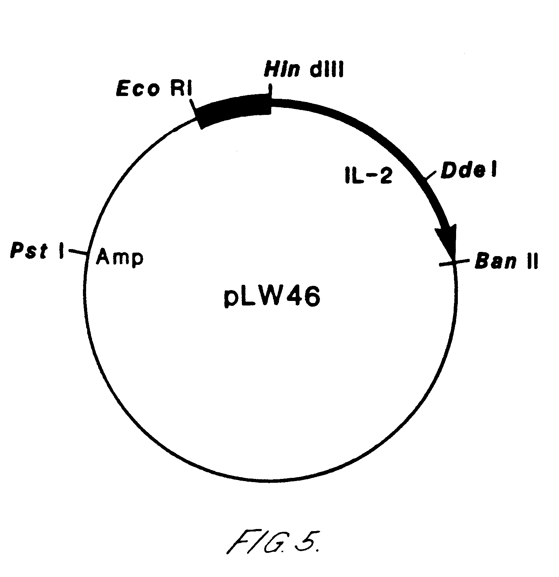

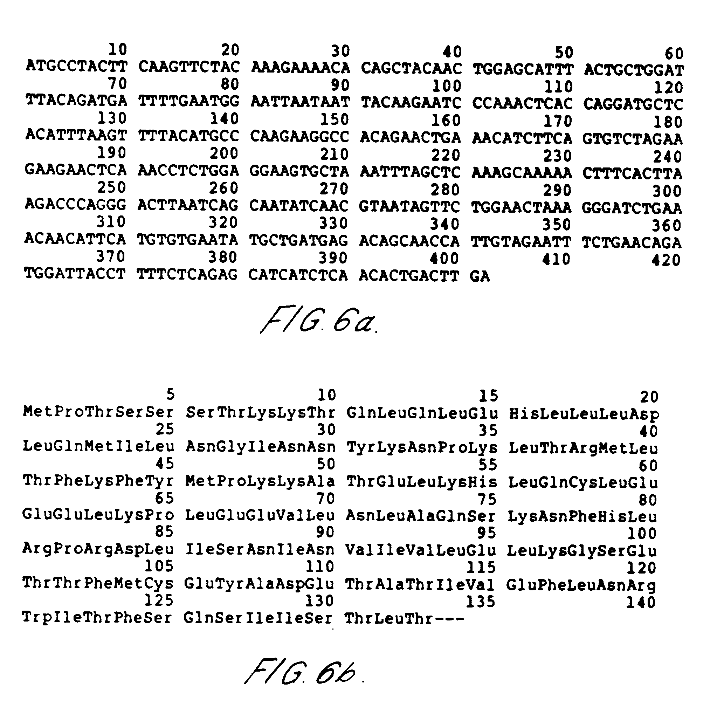

Reference is made to co-pending European Patent Application No. 83306221.9, from which the present application is divided, which application claims, inter alia, a biologically active recombinant human interleukin-2 mutein pharmaceutical or veterinary preparation suitable for use in therapeutic or diagnostic applications, which preparation is incapable of forming an improper intramolecular disulfide bridge and is stable upon storage relative to bacterially derived reduced native interleukin-2, wherein the cysteine residue normally present at position 125, numbered in accordance with native interleukin-2, that is free to form a disulfide link and is non-essential to said biological activity has been substituted by a neutral amino acid. The said co-pending application also specifically claims a human recombinant serine$_{125}$interleukin-2 mutein

which is incapable of forming an improper intramolecular disulfide bridge but which is stable upon storage relative to bacterially derived reduced native interleukin-2, and which has the deduced amino acid shown in Figure 6b herein.

The invention will now be further described and illustrated with reference to the accompanying drawings, in which:-

Figure 1 is a diagram of the cloning vector M13mp8 phage;

Figure 2 is a diagram of the expression plasmid pTrp3;

Figure 3 is a diagram of the plasmid pLW1 which contains the human interleukin-2 (IL-2) gene under the control of the E.coli trp promoter;

Figure 4 is a restriction map of phage clone M13-IL2;

Figure 5 is a restriction map of the plasmid pLW46; and

Figures 6a and 6b show, respectively, the nucleotide sequence of the coding strand of the clone pLW46 and the corresponding amino acid sequence of the IL-2 mutein designated IL-2$_{ser125}$.

In human IL-2, since the disulfide structure of the native IL-2 protein is not known, it is possible to use the present invention to create mutations at codons 58, 105 and 125 of the IL-2 gene and identify which cysteine residues are necessary for activity and therefore most likely to be involved in native disulfide bridge formation. In the same vein, the cysteine residue that is not necessary for activity can be modified so as to prevent the formation of incorrect intramolecular disulfide bridges and minimize the chance of intermolecular disulfide bridges by replacement of the free cysteine residue.

By the use of the Oligonucleotide-directed mutagenesis procedure with a synthetic oligonucleotide primer that is complementary to the region of the Il-2 gene at the codon for cys 125 but which contains single or multiple base changes in that codon, a designer gene may be produced that results in cys 125 being replaced with any other amino acid of choice.

Conversion of cys 125 to neutral amino acids such as glycine, valine, alanine, leucine, isoleucine, tyrosine, phenylalanine, histidine, tryptophan, serine, threonine and methionine is the preferred approach. Serine and threonine are the most preferred replacements because of their chemical analogy to cysteine.

The size of the oligonucleotide primer is determined by the requirement for stable hybridization of the primer to the region of the gene in which the mutation is to be induced, and by the limitations of the currently available methods for synthesizing oligonucleotides. The factors to be considered in designing oligonucleotides for use in oligonucleotide-directed mutagenesis (eg, overall size, size of portions flanking the mutation site) are described by Smith, M. and Gillam S., supra. In general the overall length of the oligonucleotide will be such as to optimize stable, unique hybridization at the mutation site with the 5′ and 3′ extensions from the mutation site being of sufficient size to avoid editing of the mutation by the exonuclease activity of the DNA polymerase. Oligonucleotides used for mutagenesis in accordance with the present invention usually contain from about 12 to about 24 bases, preferably from about 14 to about 20 bases and still more preferably from about 15 to about 18 bases. They will usually contain at least about three bases 3′ of the altered or missing codon.

Oligonucleotide-directed mutagenesis may be employed to make a mutant IL-2 gene that encodes a mutein having IL-2 activity but having cys 125 changed to serine 125. The preferred oligonucleotide primer used in making this mutant IL-2 gene when the phage carries the sense strand of the gene is GATGATGCTTCTGAGAAAAGGTAATC. This oligonucleotide has a C→G change at the middle base on the triplet that is paired with codon 125 of the IL-2 gene.

It must be recognized that the genetic code is degenerate and that many of the amino acids may be encoded by more than one codon. The base code for serine, for example, is six-way degenerate such that the codons, TCT, TCG, TCC, TCA, AGT, and ACG all code for serine. Similarly, threonine is encoded by any one of codons ACT, ACA, ACC and ACG. It is intended that when one codon is specified for a particular amino acid, it includes all degenerate codons which encode that amino acid.

The primer is hybridized to single-stranded phage such as M13, fd, or ØX174 into which a strand of the IL-2 gene has been cloned. It will be appreciated that the phage may carry either the sense strand or antisense strand of the gene. When the phage carries the antisense strand the primer is identical to the region of the sense strand that contains the codon to be mutated except for a mismatch with that codon that defines a deletion of the codon or a triplet that codes for another amino acid. When the phage carries the sense strand the primer is complementary to the region of the sense strand that contains the codon to be mutated except for an appropriate mismatch in the triplet that is paired with the codon to be deleted. Conditions that may be used in the hybridization are described by Smith, M. and Gillam, S.,. supra. The temperature will usually range between about 0°C and 70°C, more usually about 10°C to 50°C. After the hybridization, the primer is extended on the phage DNA by reaction with DNA polymerase I, T$_4$ DNA polymerase, reverse transcriptase or other suitable DNA polymerase. The resulting dsDNA is converted to closed circular dsDNA by treatment with a DNA ligase such as T$_4$ DNA ligase. DNA molecules containing singlestranded regions may be destroyed by S1 endonuclease treatment.

The resulting mutational heteroduplex is then used to transform a competent host organism or cell. Replication of the heteroduplex by the host provides progeny from both strands. Following replication the mutant gene may be isolated from progeny of the mutant strand, inserted into an appropriate expression vector, and the vector used to transform a suitable host organism or cell. Preferred vectors are plasmids pBR322, pCR1, and variants thereof, synthetic vectors and the like. Suitable host organisms are E.coli, Pseudomonas, Bacillus subtilis, Bacillus thuringiensis, various strains of yeast, Bacillus thermophilus, animal cells such as mice, rat or Chinese hamster ovary (CHO) cells, plant cells, animal and plant hosts and the like. It must be recognized that when a host of choice is transformed with the vector, appropriate promoter-operator sequences are also introduced in order for the mutein to be expressed. Hosts may be prokaryotic or eukaryotic (processes for inserting DNA into eukaryotic cells are described in PCT application nos US81/00239 and US81/00240 published 3 September 1981). E.coli and CHO cells are the preferred hosts. The muteins obtained in accordance with the present invention may be glycosylated or unglycosylated depending on the glycosylation occurring in the native parent protein and the host organism used to produce the mutein. If desired, unglycosylated mutein obtained when E.coli or a Bacillus is the host organism, may be optionally glycosylated in vitro by chemical, enzymatic and other types of modifications known in the art.

The following Examples are presented to help in the better understanding of the subject invention and for purposes of illustration only. They are not to be construed as limiting the scope of the invention.

Example 1

The nucleotide sequence for a cDNA clone coding for human IL-2, procedures for preparing IL-2 cDNA libraries, and screening same for IL-2 are described by Taniguchi, T., et al, Nature (1983) Vol 302, p 305 et seq.

cDNA libraries enriched in potential IL-2 cDNA clones were made from an IL-2 enriched mRNA fractions obtained from induced peripheral blood lymphocytes (PBL) and Jurkat cells by conventional procedures. The enrichment of the mRNA for IL-2 message was made by fractionating the mRNA and identifying the fraction having IL-2 mRNA activity by injecting the fractions in Xenopus laevis oocytes and assaying the oocyte lysates for IL-2 activity on HT-2 cells (J. Watson, J Exp Med (1979) 150:1570-1519 and S. Gillis et al, J Immun (1978) 120:2027-2032.)

Example 2

Screening and Identification of IL-2 cDNA Clones

The IL-2 cDNA libraries were screened using the colony hybridization procedure. Each microtiter plate was replicated onto duplicate nitrocellulose filter papers (S & S type BA-85) and colonies were allowed to grow at 37°C for 14-16 hr on L agar containing 50 μg/ml ampicillin. The colonies were lysed and DNA fixed to the filter by sequential treatment for 5 min with 500 mM NaOH, 1.5 M NaCl, washed twice for 5 min each time with 5 x standard saline citrate (SSC). Filters were air dried and baked at 80°C for 2 hr. The duplicate filters were pre-hybridized at 42°C for 6-8 hr with 10 ml per filter of DNA hybridization buffer (50% formamide, 5 x SSC, pH 7.0, 5 x Denhardt's solution (polyvinylpyrrolidine, plus ficoll and bovine serum albumin; 1 x = 0.2% of each), 50 mM sodium phosphate buffer at pH 7.0, 0.2% SDS, 20 μg/ml Poly U, and 50 μg/ml denatured salmon sperm DNA.

A $^{32}$p-labeled 20-mer oligonucleotide probe was prepared based on the IL-2 gene sequence reported by Taniguchi, T., et at, supra. The nucleotide sequence of the probe was GTGGCCTTCTTGGGCATGTA.

The samples were hybridized at 42°C for 24-36 hr with 5 ml/filter of DNA hybridization buffer containing the $^{32}$p cDNA probe. The filters were washed two times for 30 min each time at 50°C with 2 x SSC, 0.1% SDS, then washed twice with 1 x SSC and 0.1% SDS at 50°C for 90 min, air dried, and autoradiographed at -70°C for 2 to 3 days. Positive clones were identified and rescreened with the probe. Full length clones were identified and confirmed by restriction enzyme mapping and comparison with the sequence of the IL-2 cDNA clone reported by Taniguchi, T., et al, supra.

Example 3

Cloning of the IL-2 Gene into M13 Vector:

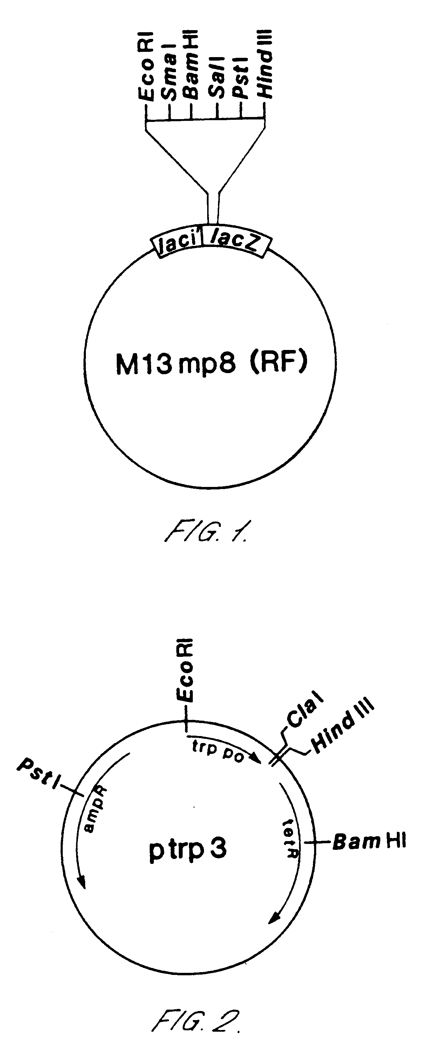

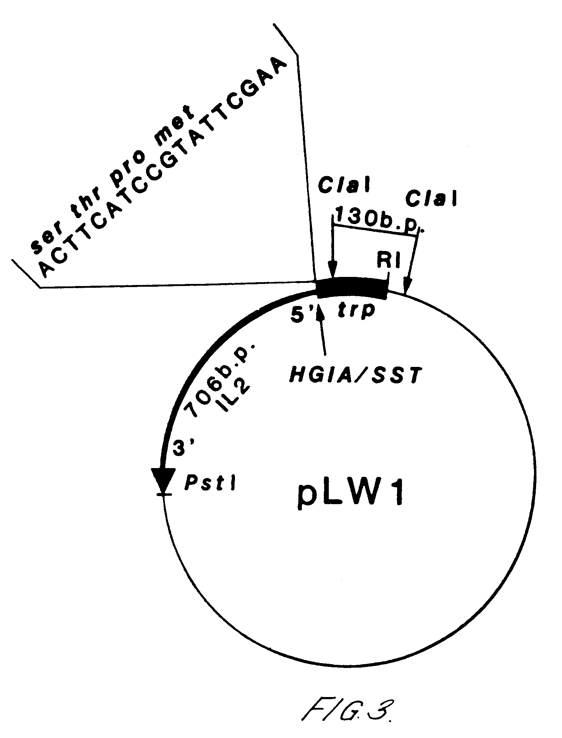

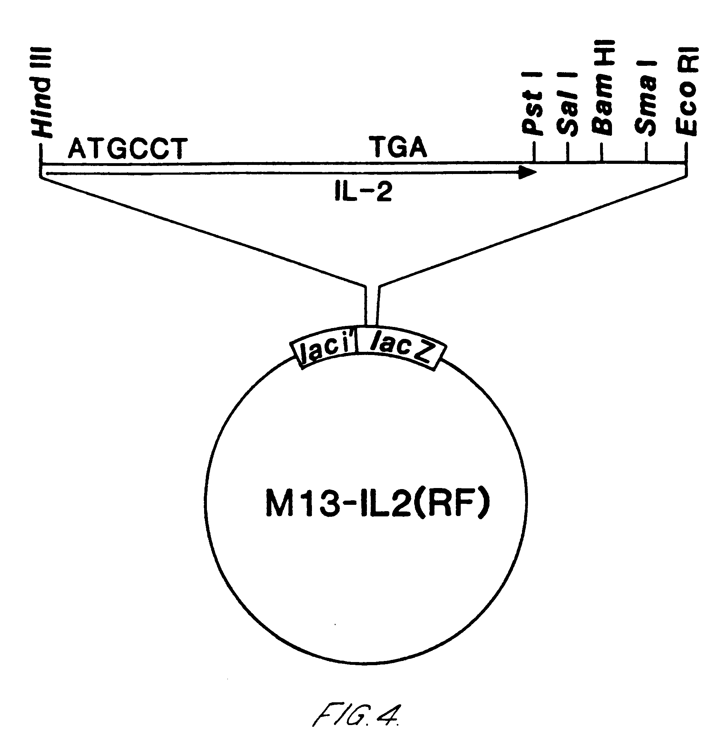

The use of M13 phage vector as a source of single-stranded DNA template has been demonstrated by G.F. Temple et al, Nature (1982) 296:537-540. Plasmid pLW1 (Figure 3) containing the IL-2 gene under control of E.coli trp promoter, was digested with the restriction enzymes HindIII and PstI. A sample of pLW1 was deposited in the American Type Culture Collection, 12301 Parklawn Drive, Rockville, Maryland 20852, USA, on 4 August 1983 and has been assigned the ATCC number 39,405. The M13mp8 (J. Messing, "Third Cleveland Symposium on Macromolecules:
Recombinant DNA." Ed. A. Walton, Elsevier Press, 143-153 (1981)) replicative form (RF) DNA (Figure 1) was digested with restriction enzymes HindIII and BamHI, and mixed with the pLW1 DNA which had previously been digested with HindIII and PstI. The mixture was then ligated with $T_4$ DNA ligase and the ligated DNA transformed into competent cells of E.coli strain JM 103 and plated on Xgal indicator plates (J. Messing, et al, Nucleic Acids Res (1981) 9:309-321). Plaques containing recombinant phage (white plaques) were picked, inoculated into a fresh culture of JM 103 and minipreps of RF molecules prepared from the infected cells (H.D. Birnboim and J. Doly, Nucleic Acid Res (1979) 7:1513-1523). The RF molecules were digested with various restriction enzymes to identify the clones containing the IL-2 insert. The restriction map of one clone (designated M13-IL2) containing the IL-2 insert is shown in Figure 4. Single-stranded (ss) phage DNA was prepared from clone M13-IL2 to serve as a template for site-specific mutagenesis using a synthetic oligonucleotide.

Example 4

Oligonucleotide-directed Mutagenesis

As indicated previously, IL-2 contains cysteine residues at amino acid positions 58, 105 and 125. Based on the nucleotide sequences of the portions of the IL-2 gene that contain the codons for these three cysteine residues three oligonucleotide primers were designed and synthesized for mutating the codons for these residues to codons for serine. These oligonucleotides have the following sequences.
CTTCTAGAGACTGCAGATGTTTC (DM27) to change cys 58,
CATCAGCATACTCAGACATGAATG (DM28) to change cys 105 and
GATGATGCTCTGAGAAAAGGTAATC (DM29) to change cys 125.
Forty picomoles of each oligonucleotide were kinased separately in the presence of 0.1 mM ATP, 50 mM Tris-HC1, pH 8.0, 10 mM $MgCl_2$, 5 mM DTT and 9 units of $T_4$ kinase in 50 $\mu$l at 37°C for 1 hr. Each of the kinased primers (10 pmoles) was hybridized to 2.6 $\mu$g of ss M13-IL2 DNA in 15 $\mu$l of a mixture containing 100 mM NaCl, 20 mM Tris-HCl, pH 7.9, 20 mM $MgCl_2$ and 20 mM $\beta$-mercaptoethanol, by heating at 67°C for 5 min and 42°C for 25 min. The annealed mixtures were chilled on ice and then adjusted to a final colume of 25 $\mu$l of a reaction mixture containing 0.5 mM of each dNTP, 17 mM Tris-HC1, pH 7.9, 17 mM $MgCl_2$, 83 mM NaCl, 17 mM $\beta$-mercaptoethanol, 5 units of DNA polymerase I Klenow fragment, 0.5 mM ATP and 2 units of $T_4$ DNA ligase, incubated at 37°C for 5 hr. The reactions were terminated by heating to 80°C and the reaction mixtures used to transform competent JM103 cells, plated onto agar plates and incubated overnight to obtain phage placques.

Example 5

Screening and Identification of Mutagenized Phage Placques

Plates containing mutagenized M13-IL2 placques as well as 2 plates containing unmutagenized M13-IL2 phage placques, were chilled to 4°C and phage placques from each plate were transferred onto two nitrocellulose filter circles by layering a dry filter on the agar plate for 5 min for the first filter and 15 min for the second filter. The filters were then placed on thick filter papers soaked in 0.2 N NaOH, 1.5 M NaCl and 0.2% Triton for 5 min, and neutralized by layering onto filter papers soaked with 0.5 M Tris-HCl, pH 7.5, and 1.5 M NaCl for another 5 min. The filters were washed in a similar fashion twice on filters soaked in 2 x SSC, dried and then baked in a vacuum oven at 80°C for 2 hr. The duplicate filters were pre-hybridized at 42°C for 4 hr with 10 ml per filter of DNA hybridization buffer (5 x SSC, pH 7.0, 4 x Denhardts solution (polyvinylpyrrolidine, ficoll and bovin serum albumin, 1x = 0.02% of each), 0.1% SDS, 50 mM sodium phosphate buffer, pH 7.0 and 100 $\mu$g/ml of denatured salmon sperm DNA. $^{32}$p-labelled probes were prepared by kinasing the oligonucleotide primers with labelled ATP. The filters were hybridized to 0.1 x 10$^5$ cpm/ml of $^{32}$p-labelled primers in 5 ml per filter of DNA hybridization buffer at 42°C for 8 hr. The filters were washed twice at 50°C for 30 min each in washing buffers containing 0.1% SDS and 2 x SSC, and twice at 50°C for 30 min each with 0.1% SDS and 0.2 x SSC. The filters were air dried and autoradiographed at -70°C for 2-3 days.

Since the oligonucleotide primers DM28 and DM29 were designed to create a new DdeI restriction site in the mutagenized clones (Figure 5), RF-DNA from a number of the clones which hybridized with each of these kinased primers were digested with the restriction enzyme DdeI. One of the mutagenized M13-IL2 placques which hybridized with the primer DM28 and has a new DdeI restriction site (M13-LW44) was picked and inoculated into a culture of JM103, ssDNA was prepared from the culture supernatant and dsRF-DNA was prepared from the cell pellet. Similarly, a placque which hybridized with primer DM29 was picked (M13-LW46) and ssDNA and RF-DNA prepared from it. The oligonucleotide primer DM27 was designed to create a new PstI restriction site instead of a DdeI site. Therefore, the placques that hybridized to this primer were screened for the presence of a new PstI site. One such phage placque was identified (M13-LW42) and ssDNA and RF-DNA prepared from it. The DNA from all three of these clones were sequenced to confirm that the target TGT codons for cysteine had been converted to a TCT codon for serine.

### Example 6

### Recloning of the Mutagenized IL-2 Gene for Expression in E.coli

RF-DNA from M13-LW42, M13-LW44 and M13-LW46 were each digested with restriction enzymes HindIII and BanII and the insert fragments purified from a 1% agarose gel. Similarly, the plasmid pTrp3 (Figure 2) was digested with HindIII and BanII, the large plasmid fragment containing the trp promoter was purified on an agarose gel and then ligated with each of the insert fragments isolated from M13-LW42, M13-LW44 and M13-LW46. The ligated plasmids were transformed into competent E.coli K12 strain MM294. The plasmid DNAs from these transformants were analysized by restriction enzyme mapping to confirm the presence of the plasmids pLW42, pLW44 and pLW46. Figure 5 is a restriction map of pLW46. When each of these individual clones were grown in the absence of tryptophane to induce the trp promoter and cell free extracts analyzed on SDS-polyacrylamide gels, all three clones, pLW42, pLW44 and pLW46, were shown to synthesize a 14.5 kd protein similar to that found in the positive control, pLW21, which has been demonstrated to synthesize a 14.4 kd IL-2 protein. When these-same extracts were subjected to assay for IL-2 activity on mouse HT-2 cells, only clones pLW21 (positive control) and pLW46 had significant amounts of IL-2 activity (Table below), indicating that cys 58 and cys 105 are necessary for biological activity and changing them to serines (pLW42 and pL444 respectively) resulted in the loss of biological activity. Cys 125 on the other hand must not he necessary for biological activity because changing it to ser 125 (pLW46) did not affect the biological activity.

Table

| Clones | IL-2 Activity ($\mu$/ml) |
|---|---|
| pIL2-7 (negative control) | 1 |
| pLW21 (positive control) | 113,000 |
| pLW42 | 660 |
| pLW44 | 1,990 |
| pLW46 | 123,000 |

Figure 6a shows the nucleotide sequence of the coding strand of clone pLW46. As compared to the coding strand of the native human IL-2 gene clone pLW46 has a single base change of G → C at nucleotide 374. Figure 6b shows the corresponding amino acid sequence of the IL-2 mutein encoded by pLW46. This mutein is designated IL-2$_{ser125}$ As compared to native IL-2 the mutein has a serine instead of a cysteine at position 125.

A sample of E.coli K12 strain MM294 transformed with pLW46 was deposited in the American Type Culture Collection, 12301 Parklawn Drive, Rockville, Maryland 20852, USA on 26 September 1983 and has been assigned ATCC Number 39,452.

Muteins of IL-2 in which the cysteine at position 125 has been replaced with another amino acid, such as the mutein IL-2$_{ser 125}$ retain IL-2 activity. They may, therefore, be formulated and used in the same manner as native IL-2. Accordingly, such IL-2 muteins are useful in the diagnosis and treatment of bacterial, viral, parasitic, protozoan and fungal infections; in manifestations of lymphokine or immunodeficiency; for reconstitution of normal immunofunction in aged humans and animals; in the development of diagnostic assays such as those employing enzyme amplification, radiolabelling, radioimaging, and other methods known in the art for monitoring IL-2 levels in the diseased state; for the promotion of T cell growth in vitro for therapeutic and diagnostic purposes for blocking receptor sites for lymphokines; and in various other therapeutic, diagnostic and research applications. The various therapeutic and diagnostic applications of human IL-2 have been investigated and reported in S.A. Rosenberg, E.A. Grimm, et al, A. Mazumder, et al., and E.A. Grimm and S.A. Rosenberg. IL-2 muteins may be used by themselves or in combination with other immunologically relevant B or T cells or other therapeutic agents. For therapeutic or diagnostic applications (whether for human or veterinary use), they may, for example, be formulated in non-toxic, nonallergenic, physiolocally compatable carrier media such as distilled water, Ringer's solution, Hank's solution, physiological saline and the like. Administration of the IL-2 muteins to humans or animals may be oral or intraperitoneal or intramuscular or subcutaneous as deemed appropriate by the physician. Examples of relevant cells are B or T cells, natural killer cells, and the like and exemplary therapeutic reagents which may be used in combination with the polypeptides of this invention are the various interferons, especially gamma interferon, B cell growth factor, IL-1 and the like.

Pharmaceutical or veterinary formulations comprising the present muteins are claimed in general (as is the preparation of the said muteins) in the aforesaid co-pending European Patent Application No. 83306221.9.

## Claims

1. A biologically active recombinant mutein of human interleukin-2, which mutein is incapable of forming an improper intramolecular disulfide bridge and is stable upon storage relative to bacterially derived reduced native interleukin-2, wherein the cysteine residue normally present at position 125, numbered in accordance with native interleukin-2, that is free to form a disulfide link and is non-essential to said biological activity has been substituted by a neutral amino acid, said mutein being a threonine$_{125}$ interleukin-2 mutein, glycine$_{125}$ interleukin-2 mutein, a valine$_{125}$ interleukin-2 mutein, a leucine$_{125}$ interleukin-2 mutein, and isoleucine$_{125}$ interleukin-2 mutein, a histidine$_{125}$ interleukin-2 mutein, a tyrosine$_{125}$ interleukin-2 mutein, a phenylalanine$_{125}$ interleukin-2 mutein, a tryptophan$_{125}$ interleukin-2 mutein, or a methionine$_{125}$ interleukin-2 mutein.

2. A mutein as claimed in claim 1 and which is unglycosylated.

3. A DNA sequence encoding a mutein as defined in claim 1.

4. An expression vector that includes a DNA sequence as defined in claim 3.

5. E.coli tranformed with an expression vector as defined in claim 4.

6. The use of an expression vector as defined in Claim 4 in making a recombinant interleukin-2 mutein by expressing said vector.

7. A method of making a DNA sequence as defined in Claim 3 comprising:
   (a) hybridizing a single-stranded DNA comprising a strand of a gene that encodes for an interleukin-2 protein with a mutant oligonucleotide primer that is complementary to a region of said strand that includes the codon for said cysteine residue or the antisense triplet paired with said codon, as the case may be, except for a mismatch with said codon or said antisense triplet which mismatch defines a triplet that codes for said neutral animo acid;
   (b) extending the primer with DNA polymerase to form a mutational heteroduplex; and
   (c) replicating said mutational heteroduplex.

## Revendications

1. Mutéine recombinante biologiquement active dérivée de l'interleukine-2 humaine, cette mutéine étant incapable de former un pont disulfure intramoléculaire non souhaitable, et étant stable lors de sa conservation par rapport à l'interleukine-2 native réduite d'origine bactérienne, dans laquelle le résidu cystéine normalement présent à la position 125 numérotée selon l'interleukine-2 native, qui peut former une liaison disulfure et qui n'est pas essentiel à ladite activité biologique, a été substitué par un aminoacide neutre, cette mutéine étant une thréonine$_{125}$ interleukine-2 mutéine, une glycine$_{125}$ interleukine-2 mutéine, une valine$_{125}$-interleukine-2 mutéine, une leucine$_{125}$ interleukine mutéine, et une

isoleucine$_{125}$interleukine-2 mutéine, une histidine$_{125}$interleukine-2 mutéine, une tyrosine$_{125}$interleukine-2 mutéine, une phénylalanine$_{125}$interleukine-2 mutéine, une tryptophane$_{125}$interleukine-2 mutéine, ou une méthionine$_{125}$interleukine mutéine.

**2.** Mutéine selon la revendication 1, qui est non glycosylée.

**3.** Séquence d'ADN codant pour une mutéine selon la revendication 1.

**4.** Vecteur d'expression, comprenant une séquence d'ADN selon la revendication 3.

**5.** E. coli transformé avec un vecteur d'expression selon la revendication 4.

**6.** Utilisation d'un vecteur d'expression selon la revendication 4, pour produire une mutéine recombinante d'interleukine-2, par expression de ce vecteur.

**7.** Procédé de préparation d'une séquence d'ADN selon la revendication 3, dans lequel :
(a) on hybride un ADN monocaténaire comprenant un brin d'un gène codant pour une protéine interleukine-2, avec une amorce oligonucléotidique mutante complémentaire d'une région du brin qui comprend le codon correspondant au résidu cystéine ou, ainsi que cela peut être le cas, au triplet anti-sens apparié avec ce codon, à l'exception d'une erreur d'appariement avec ce codon ou le triplet anti-sens, cette erreur d'appariement définissant un triplet qui code pour ledit aminoacide neutre ;
(b) on allonge l'amorce avec une ADN polymérase, afin de former une double hélice hétérogène mutante ; et
(c) on réplique la double hélice hétérogène mutante.

## Patentansprüche

**1.** Biologisch aktives rekombinantes Mutein von menschlichem Interleukin-2, wobei das Mutein nicht in der Lage ist, eine ungeeignete intramolekulare Disulfidbrücke zu bilden, und das Mutein bei Lagerung stabil ist, bezogen auf bakteriell hergeleitetes, reduziertes, natürliches Interleukin-2, wobei der normalereise in Position 125 vorliegende Cysteinrest, numeriert gemäß dem natürlichen Interleukin-2, der zur Bildung einer Disulfidbrücke frei und für die biologische Aktivität nicht essentiell ist, durch eine neutrale Aminosäure ersetzt worden ist, wobei das Mutein ein Threonin$_{125}$-Interleukin-2-Mutein, ein Glycin$_{125}$-Interleukin-2-Mutein, ein Valin$_{125}$-Interleukin-2-Mutein, ein Leucin$_{125}$-Interleukin-2-Mutein und Isoleucin$_{125}$-Interleukin-2-Mutein, ein Histidin$_{125}$-Interleukin-2-Mutein, ein Tyrosin$_{125}$-Interleukin-2-Mutein, ein Phenylalanin$_{125}$-Interleukin-2-Mutein, ein Tryptophan$_{125}$-Interleukin-2-Mutein oder ein Methionin$_{125}$-Interleukin-2-Mutein darstellt.

**2.** Mutein nach Anspruch 1, das unglycosyliert ist.

**3.** DNA-Sequenz, die ein Mutein nach Anspruch 1 codiert.

**4.** Expressionsvektor, der eine DNA-Sequenz nach Anspruch 3 umfaßt.

**5.** E. coli, das mit einem Expressionsvektor nach Anspruch 4 transformiert ist.

**6.** Verwendung eines Expressionsvektors nach Anspruch 4 zur Herstellung eines rekombinanten Interleukin-2-Muteins durch Expression des Vektors.

**7.** Verfahren zur Herstellung einer DNA-Sequenz nach Anspruch 3, umfassend:
(a) Hybridisierung einer einzelsträngigen DNA, umfassend einen Strang eines Gens, das für ein Interleukin-2-Protein codiert, mit einem mutierten Oligonucleotid-Primer, der zu einem Bereich des Strangs komplementär ist, der das Codon für den Cysteinrest oder je nachdem das mit dem Codon gepaarte Antisens-Triplett umfaßt, mit Ausnahme einer Fehlpaarung mit dem Codon oder dem Antisens-Triplett, wobei die Fehlpaarung ein Triplett definiert, das die neutrale Aminosäure codiert;
(b) Verlängerung des Primers mit DNA-Polymerase zur Bildung einer mutierten Heteroduplex; und
(c) Replikation der mutierten Heteroduplex.

FIG. 1.

FIG. 2.

FIG. 3.

FIG. 4.

FIG. 5.

```
           10         20         30         40         50         60
ATGCCTACTT CAAGTTCTAC AAAGAAAACA CAGCTACAAC TGGAGCATTT ACTGCTGGAT
           70         80         90        100        110        120
TTACAGATGA TTTTGAATGG AATTAATAAT TACAAGAATC CCAAACTCAC CAGGATGCTC
          130        140        150        160        170        180
ACATTTAAGT TTTACATGCC CAAGAAGGCC ACAGAACTGA AACATCTTCA GTGTCTAGAA
          190        200        210        220        230        240
GAAGAACTCA AACCTCTGGA GGAAGTGCTA AATTTAGCTC AAAGCAAAAA CTTTCACTTA
          250        260        270        280        290        300
AGACCCAGGG ACTTAATCAG CAATATCAAC GTAATAGTTC TGGAACTAAA GGGATCTGAA
          310        320        330        340        350        360
ACAACATTCA TGTGTGAATA TGCTGATGAG ACAGCAACCA TTGTAGAATT CTGAACAGA
          370        380        390        400        410        420
TGGATTACCT TTTCTCAGAG CATCATCTCA ACACTGACTT GA
```

*FIG. 6a.*

```
                 5                 10                 15                 20
MetProThrSerSer SerThrLysLysThr GlnLeuGlnLeuGlu HisLeuLeuLeuAsp
                25                 30                 35                 40
LeuGlnMetIleLeu AsnGlyIleAsnAsn TyrLysAsnProLys LeuThrArgMetLeu
                45                 50                 55                 60
ThrPheLysPheTyr MetProLysLysAla ThrGluLeuLysHis LeuGlnCysLeuGlu
                65                 70                 75                 80
GluGluLeuLysPro LeuGluGluValLeu AsnLeuAlaGlnSer LysAsnPheHisLeu
                85                 90                 95                100
ArgProArgAspLeu IleSerAsnIleAsn ValIleValLeuGlu LeuLysGlySerGlu
               105                110                115                120
ThrThrPheMetCys GluTyrAlaAspGlu ThrAlaThrIleVal GluPheLeuAsnArg
               125                130                135                140
TrpIleThrPheSer GlnSerIleIleSer ThrLeuThr---
```

*FIG. 6b.*